# EUROPEAN PATENT APPLICATION

(11) **EP 3 434 198 A1**
(43) Date of publication of application: **30.01.2019**
(21) Application number: 17769882.6
(22) Date of filing: 06.03.2017
(51) Int. Cl.: A61B 17/062, A61B 17/29

(54) **COOLING DEVICE**

(30) Priority: 23.03.2016 JP 2016059170
(71) Applicant: Yoshimi Inc., Obu-shi, Aichi 474-0038 (JP); Akiyama Medical Co., Ltd., Tokyo 113-0033 (JP); Kyushu University, National University Corporation, Fukuoka-shi, Fukuoka 812-8581 (JP)
(72) Inventor: YOSHIMI Yukiharu, Obu-shi, Aichi 474-0038 (JP); KAWAI Hirohisa, Tokyo 113-0033 (JP); IKEDA Tetsuo, Fukuoka-shi, Fukuoka 812-8581 (JP)
(74) Representative: Patentanwälte Bals & Vogel
(86) International application number: PCT/JP2017/008765
(87) International publication number: WO 2017/163837

(57) **Abstract**

A cooling device 10 may be configured to cool a shape-memory alloy member. The cooling device 10 may include: a cooling unit 20 configured to cool the shape-memory alloy member or a part that is to make contact with the shape-memory alloy member; a refrigerant supply pipe 30, 31 including one end connected to a refrigerant supply source 34 and other end connected to the cooling unit 20, the refrigerant supply pipe 30, 31 configured to supply a refrigerant supplied from the refrigerant supply source 34 to the cooling unit 20; and a refrigerant discharge pipe 32, 33 including one end connected to the cooling unit 20, the refrigerant discharge pipe 32, 33 configured to discharge the refrigerant discharged from the cooling unit 20 from other end of the refrigerant discharge pipe 32, 33.

## Description

### Technical Field

The technique disclosed herein relates to a cooling device. Specifically, it relates to a cooling device configured to cool a shape-memory alloy member.

### Background Art

A shape memory alloy is known to change its material characteristic according to a temperature change. By making use of this change in the material characteristic, applications of the shape memory alloy to various products and components are being considered. For example, medical apparatuses and actuators using the shape memory alloy are being considered. An actuator using the shape memory alloy is described, for example, in Patent Literature 1.

### Citation List

### Patent Literature

Patent Literature 1: Japanese Patent Application Publication No. 2015-206331

### Summary

To make use of characteristics of a shape memory alloy, a temperature of the shape memory alloy needs to be controlled. That is, a material characteristic thereof needs to be changed by heating the shape memory alloy or by cooling the shape memory alloy. In a case of heating the shape memory alloy, such heating can be performed by a simple method such as electrical heating. On the other hand, although several methods are considered as methods of cooling the shape memory alloy, they have various problems. For example, a simple cooling method may be to cool the shape memory alloy by immersing it in a refrigerant such as cooling water, however, there may be cases where it is difficult to immerse the shape memory alloy in the refrigerant depending on an environment in which a component constituted of the shape memory alloy is used. Further, in a case where a transformation point of the shape memory alloy is close to or lower than 0°C, it is difficult to use water as cooling means. Further, since a transformation speed of the shape memory alloy is slowed down when a temperature of the cooling water and a transformation temperature of the shape memory alloy are close, there is a need to increase a temperature difference therebetween to a certain degree. The description herein discloses a technique that can suitably cool a shape-memory alloy member.

A cooling device disclosed herein may be configured to cool a shape-memory alloy member. The cooling device may comprise: a cooling unit configured to cool the shape-memory alloy member or a part that is to make contact with the shape-memory alloy member; a refrigerant supply pipe including one end connected to a refrigerant supply source and other end connected to the cooling unit, the refrigerant supply pipe configured to supply a refrigerant supplied from the refrigerant supply source to the cooling unit; and a refrigerant discharge pipe including one end connected to the cooling unit, the refrigerant discharge pipe configured to discharge the refrigerant discharged from the cooling unit from other end of the refrigerant discharge pipe.

In the above cooling device, the refrigerant supplied from the refrigerant supply source is supplied to the cooling unit through the refrigerant supply pipe and is discharged from the cooling unit through the refrigerant discharge pipe. Due to this, the refrigerant does not leak to outside of the cooling device since the refrigerant was supplied to the cooling unit until discharged therefrom. Further, the shape-memory alloy member is cooled by the cooling unit which is cooled by the refrigerant. Due to this, the shape-memory alloy member can easily be cooled by using the refrigerant, without being immersed in the refrigerant.

### Brief Description of Drawings

FIG. 1 is a diagram showing a schematic configuration of a cooling device according to an embodiment;
FIG. 2 is a cross-sectional view of a primary part II of FIG. 1;
FIG. 3 is a cross-sectional view of a distal end portion of gripping forceps according to the embodiment;
FIG. 4 is a diagram explaining a method of using the cooling device, and is a diagram showing a state before gripping forceps are inserted to a guide pipe;
FIG. 5 is a diagram explaining the method of using the cooling device, and is an enlarged view of a primary part V of FIG. 4;
FIG. 6 is a diagram explaining the method of using the cooling device, and is a diagram showing a state in which the gripping forceps are inserted to the guide pipe;
FIG. 7 is a diagram explaining the method of using the cooling device, and is a diagram showing a state in which the cooling device is inserted to the guide pipe; and
FIG. 8 is a diagram showing a schematic configuration of a cooling device according to another embodiment.

### Description of Embodiments

Some of the features characteristic to below-described embodiments will herein be listed. It should be noted that the respective technical elements are independent of one another, and are useful solely or in combinations. The combinations thereof are not limited to those described in the claims as originally filed.
(Feature 1) The cooling device disclosed herein may further comprise a first refrigerant accommodating space connected to the other end of the refrigerant discharge pipe. The first refrigerant accommodating space may be configured to accommodate the refrigerant discharged from the refrigerant discharge pipe. According to such a configuration, the refrigerant discharged from the refrigerant discharge pipe is accommodated in the first refrigerant accommodating space, so the refrigerant can be prevented from making contact with surrounding devices, a body, and the like.
(Feature 2) cooling device disclosed herein, the cooling unit may comprise: a cooling surface configured to cool the shape-memory alloy member or a part that is to make contact with the shape-memory alloy member; and a second refrigerant accommodating space configured to accommodate the refrigerant for cooling the cooling surface. The refrigerant supplied from the refrigerant supply source may be supplied to the second refrigerant accommodating space through the refrigerant supply pipe, and the refrigerant supplied to the second refrigerant accommodating space may be discharged through the refrigerant discharge pipe. According to such a configuration, the cooling unit comprises the second refrigerant accommodating space configured to accommodate the refrigerant supplied from the refrigerant supply source. Due to this, the cooling surface can suitably be cooled, and the shape-memory alloy member can suitably be cooled via the cooling surface.
(Feature 3) In the cooling device disclosed herein, the second refrigerant accommodating space may be covered with a heat insulating material excluding a cooling surface side thereof. According to such a configuration, heat can be prevented from transferring to the refrigerant inside the second refrigerant accommodating space from outside other than the cooling surface. Due to this, the cooling surface can be cooled efficiently, and regions other than the cooling surface can be avoided from being cooled unnecessarily.
(Feature 4) In the cooling device disclosed herein, the cooling surface may be constituted of copper, copper alloy, aluminum, aluminum alloy, or metal having higher thermal conductivity than aluminum. According to such a configuration, a thermal conductivity of the cooling surface can be increased, and the shape-memory alloy member can suitably be cooled.
(Feature 5) The cooling device disclosed herein may further comprise an operation handle configured to control a supply state and a non-supply state, wherein the supply state is a state where the refrigerant is supplied from the refrigerant supply source to the second refrigerant accommodating space, and the non-supply state is a state where the refrigerant is not supplied from the refrigerant source to the second refrigerant accommodating space. According to such a configuration, cooling capacity of the cooling surface can be adjusted by the handle, so a temperature of the shape-memory alloy member can suitably be adjusted.
(Feature 6) The cooling device disclosed herein may further comprise a guide pipe configured to accommodate the refrigerant supply pipe and the refrigerant discharge pipe, and to guide the refrigerant supply pipe and the refrigerant discharge pipe to the cooling unit. The cooling unit may comprise a tubular member connected to one end of the guide pipe, and the tubular member may be provided with the cooling surface on a surface thereof and be provided with the second refrigerant accommodating space inside thereof. According to such a configuration, the cooling device is configured of the elongate tubular member, and thus the cooling device can suitably be inserted to a narrow space.

### Embodiments

Hereinbelow, a cooling device 10 according to an embodiment will be described with reference to the drawings. The cooling device 10 is configured to cool a suture needle 70 used in an endoscopic surgery. The suture needle 70 is constituted of a shape memory alloy, and the cooling device 10 is used to cool the suture needle 70 (which is an example of a medical instrument) inside a body of a patient. In recent surgical procedures, an endoscopic surgery is broadly practiced in order to lessen traumatic insult on patients. The endoscopic surgery does not create a large incision on a patient's body, but it rather creates a small incision on the patient's body and inserts an endoscope and another surgical instrument through the created small incision into the patient's body to be performed therein. Due to this, when a surgical instrument is inserted to the patient's body, it needs to be inserted through the small incision created on the patient's body, thus a shape of the surgical instrument is restricted by a size of the incision created on the patient's body. Therefore, use of the suture needle 70 constituted of the shape memory alloy is being considered.

Shape memory alloy is subjected to a shape-memory thermal treatment, and the shape memory alloy is thereby endowed with a shape memory characteristic that allows it to deform relatively freely at a predetermined temperature (that is, a transformation point) or lower to take a desired shape, as well as allows it to be restored to its originally "memorized" shape at the predetermined temperature (the transformation point) or higher. Generally, a temperature in a patient's body is higher than a temperature outside the body. Due to this, by setting the transformation point of the shape memory alloy to be the transformation point (such as 35°C) or higher inside the body and to be the transformation point (such as 35°C) or lower outside of the body, the suture needle 70 can be restored to its original shape when it is inserted into the body. Thus, according to the suture needle 70 making use of the shape memory characteristic, it is possible to deform the suture needle 70 into a desired shape outside the body since the outside of the patient's body is at the predetermined temperature or lower. For example, the suture needle 70 may be cooled to reduce its temperature by using a cooling device outside the patient's body, and the cooled suture needle 70 may be deformed by a martensite transformation. Due to this, the suture needle 70 can be deformed outside the patient's body to a shape that can easily be inserted into the body, before inserted into the patient's body. For example, the suture needle 70 can be given a shape that can easily be inserted into the patient's body by being deformed into a ring shape or a linear shape. Due to this, the relatively large suture needle 70 can be inserted into the patient's body safely and fast. Further, according to the suture needle 70 making use of the shape memory characteristic, since the inside of the patient's body is at the predetermined temperature or higher, the suture needle 70 experiences reverse transformation when its temperature rises upon being inserted into the patient's body and is restored to its original shape. Due to this, a surgical operation can be performed inside the patient's body. As above, according to the suture needle 70 making use of the shape memory characteristic, since the suture needle 70 is capable of deforming outside the patient's body upon a surgery and of being restored to the original shape inside the patient's body, the relatively large suture needle 70 can be inserted into the patient's body safely and fast, and a surgical operation can be performed therein. On the other hand, when taken out from the patient's body after the surgical operation, the suture needle 70 making use of the shape memory characteristic needs to be re-deformed to a shape that can easily be taken out from the patient's body. That is, the suture needle 70 needs to be cooled inside the patient's body to reduce its temperature, and the cooled suture needle 70 needs to be deformed by martensite transformation. However, since a refrigerant, such as cooling gas or water, cannot be injected directly into the patient's body, it is difficult to cool the suture needle 70 by making the refrigerant directly contact therewith. Although cooling the suture needle 70 by using means other than the refrigerant, for example, by using a Peltier element, may be considered, the Peltier element needs to be inserted through an incision created on the patient's body as aforementioned, thus a size of the Peltier element is restricted by the incision created on the patient's body. As a result, it is difficult to have it configured to exhibit a sufficient cooling capacity. Further, in the case of using the Peltier element, an opposite surface of the Peltier element generates heat during cooling, and thus there is a risk that the heated surface accidentally makes contact with a tissue in the patient's body. As above, it was difficult to cool the suture needle 70 inside the patient's body.

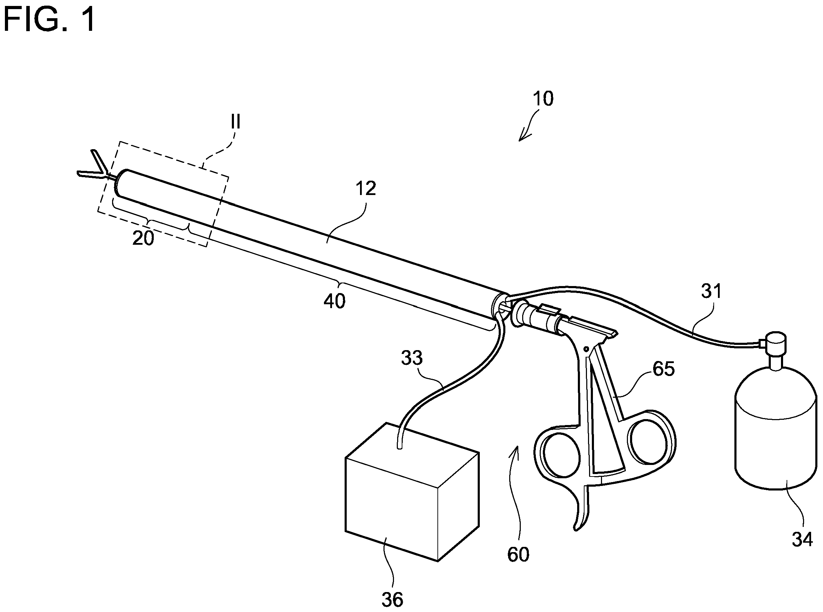

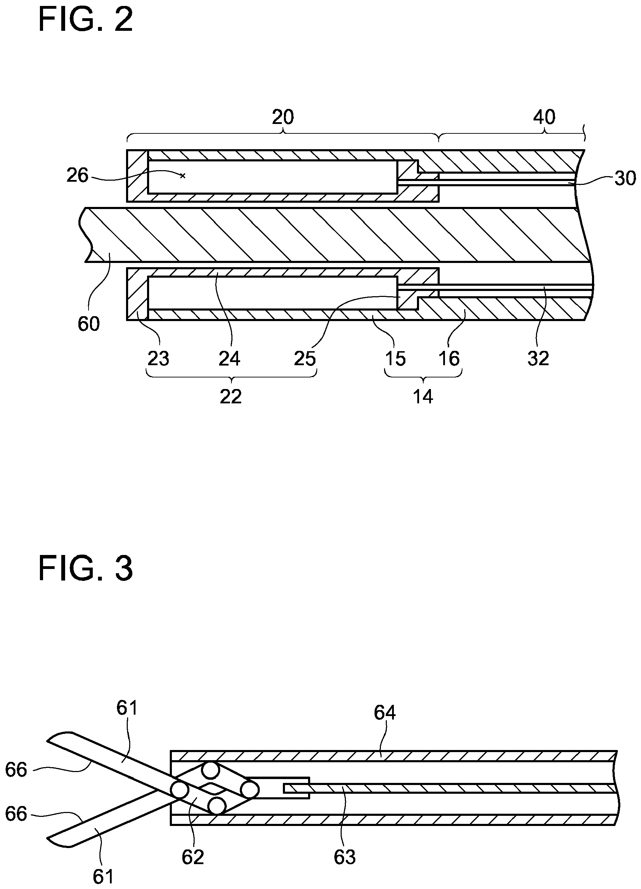

The cooling device 10 of the present embodiment is used to deform the suture needle 70 into that can easily be taken out from the patient's body when the suture needle 70 is taken out of the patient's body after an endoscopic surgery. As shown in FIGS. 1 and 2, the cooling device 10 includes a first tubular member 12, a refrigerant supply source 34, a refrigerant accommodating container 36, and a supply pipe 30 and a discharge pipe 32 that are arranged in the first tubular member 12. Inside of the first tubular member 12 is hollow, and the internal space thereof extends from a proximal end to a distal end thereof. Gripping forceps 60 can be inserted into the first tubular member 12. That is, the gripping forceps 60 can penetrate the internal space of the first tubular member 12 and can be inserted from the proximal end of the first tubular member 12. The refrigerant supply source 34 is connected to the supply pipe 30 arranged in the first tubular member 12 via a tube 31. The refrigerant accommodating container 36 is connected to the discharge pipe 32 arranged in the first tubular member 12 via a tube 33.

The first tubular member 12 has a cylindrical shape, extends along an axial direction of the gripping forceps 60, and is arranged around an outer periphery of the gripping forceps 60. The first tubular member 12 includes a cooling unit 20 arranged on a distal side and a guide unit 40 arranged on a proximal side. As shown in FIG. 2, the first tubular member 12 includes a tube 14 arranged on an outer periphery thereof and a second tubular member 22 attached to a distal portion 15 of the tube 14. The tube 14 is constituted of a heat insulating material having a low thermal conductivity, and includes the distal portion 15 and a proximal portion 16. A thickness of the distal portion 15 in a radial direction is set to be smaller than a thickness of the proximal portion 16 in the radial direction. In the description below, "thickness" of a cylindrical member refers to its dimension in the radial direction. The distal portion 15 is arranged in the cooling unit 20. The proximal portion 16 is arranged in the guide unit 40. The supply pipe 30 and the discharge pipe 32 are arranged inside the first tubular member 12. The gripping forceps 60 penetrate through inside of the first tubular member 12.

The cooling unit 20 is arranged on the distal side of the first tubular member 12. The cooling unit 20 includes the distal portion 15 of the tube 14, a part of the proximal portion 16 of the tube 14, and the second tubular member 22. The cooling unit 20 is provided with a space 26 surrounded by the second tubular member 22 and the tube 14. On an inner side relative to an inner peripheral surface of the cooling unit 20, the gripping forceps 60 penetrate the cooling unit 20.

An outer periphery of the cooling unit 20 includes the distal portion 15 and a part of the proximal portion 16 of the tube 14. A portion of the tube 14 that is arranged in the cooling unit 20 has a small thickness on the distal side and has a large thickness only at a part thereof on the proximal side. Since the tube 14 is constituted of the heat insulating material, heat can be prevented from transferring to the space 26 in the cooling unit 20 from outside. Further, since the tube 14 is constituted of the heat insulating material, in a case of using the cooling device 10 in a surgery on a human body such as an endoscopic surgery for example, a chilling injury such as frostbite can be prevented even when the tube 14 makes direct contact with the body.

The second tubular member 22 has a cylindrical shape and is arranged on an inner peripheral side of the tube 14. The second tubular member 22 is constituted of metal having a high thermal conductivity, and for example, may be constituted of copper, copper alloy, aluminum, aluminum alloy, or metal with a higher thermal conductivity than aluminum. The second tubular member 22 includes a distal portion 23, a proximal portion 25, and an intermediate portion 24. Inner diameters of the distal portion 23, the proximal portion 25, and the intermediate portion 24 are identical. On the inner side relative to an inner peripheral surface of the second tubular member 22, the gripping forceps 60 penetrate. An outer peripheral surface of the second tubular member 22 is in contact with the space 26. Since the second tubular member 22 is in contact with the space 26, the second tubular member 22 is cooled when refrigerant is supplied into the space 26.

The distal portion 23 of the second tubular member 22 has a ring shape and is joined to the distal portion 15 of the tube 14 at an outer peripheral end thereof. The distal portion 23 of the second tubular member 22 and the distal portion 15 of the tube 14 are, for example, joined by welding or configured as an integrated tube member, and an interface therebetween is sealed. An inner peripheral end of the distal portion 23 is joined to the intermediate portion 24. The distal portion 23 and the intermediate portion 24 are, for example, joined by welding or configured as an integrated tube member, and an interface therebetween is sealed. A thickness of the distal portion 23 is set to be thicker than a thickness of the intermediate portion 24 and the thickness of the distal portion 15 of the tube 14. An outer diameter of the distal portion 23 is identical to an outer diameter of the distal portion 15 of the tube 14, and the inner diameter of the distal portion 23 is identical to the inner diameter of the intermediate portion 24.

The intermediate portion 24 of the second tubular member 22 has a cylindrical shape and is arranged between the distal portion 23 and the proximal portion 25. The intermediate portion 24 and the distal portion 23 are, for example, joined by welding or configured as an integrated tube member, and the interface therebetween is sealed. The intermediate portion 24 and the proximal portion 25 are, for example, jointed by welding or configured as an integrated tube member, and an interface therebetween is sealed. The thickness of the intermediate portion 24 is set to be thinner than the thickness of the distal portion 23 and the thickness of the proximal portion 25. Due to this, an outer diameter of the intermediate portion 24 is smaller than the outer diameter of the distal portion 23 and an outer diameter of the proximal portion 25. On the other hand, the inner diameter of the intermediate portion 24 is identical to the inner diameter of the distal portion 23 and the inner diameter of the proximal portion 25. An axis line of the intermediate portion 24 is identical to an axis line of the distal portion 15, and the second tubular member 22 and the tube 14 are arranged coaxially. The intermediate portion 24 is arranged on the inner side relative to the distal portion 15. The space 26 is provided between the intermediate portion 24 and the distal portion 15.

The proximal portion 25 of the second tubular member has ring shape, and the intermediate portion 24 is joined to an inner peripheral end thereof. The proximal portion 25 is in contact with a distal end surface of the proximal portion 16 and an inner peripheral surface of the distal portion 15 of the tube 14, and interfaces therebetween are sealed by being joined by welding. The thickness of the proximal portion 25 is set to be thicker than the thickness of the intermediate portion 24. A distal end surface of the proximal portion 25 is in contact with the space 26. The supply pipe 30 and the discharge pipe 32 penetrate inside the proximal portion 25 along the axial direction.

The space 26 is provided between the tube 14 and the second tubular member 22. Specifically, the space 26 is configured by being surrounded by the distal portion 23, the intermediate portion 24, and the proximal portion 25 of the second tubular member 22 and the distal portion 15 of the tube 14. The space 26 communicates with the supply pipe 30 and the discharge pipe 32 on the proximal side. As aforementioned, each region where the second tubular member 22 and the tube 14 are in contact with is sealed by welding, so the space 26 communicates only with the supply pipe 30 and the discharge pipe 32. Due to this, the refrigerant supplied from the supply pipe 30 is accommodated in the space 26, and is discharged from the discharge pipe 32.

The guide unit 40 is arranged on the proximal side of the first tubular member 12 and includes the proximal portion 16 of the tube 14. The thickness of the proximal portion 16 of the tube 14 is set to be thicker than the thickness of the distal portion 15 on the distal side thereof. The guide unit 40 accommodates the supply pipe 30 and the discharge pipe 32. The gripping forceps 60 penetrate inside the guide unit 40.

The supply pipe 30 is constituted of metal and is arranged along the axial direction inside the guide unit 40. A distal end of the supply pipe 30 penetrates the proximal portion 25 of the second tubular member 22 and is communicated with the space 26. The supply pipe 30 and the proximal portion 25 of the second tubular member 22 are joined. A proximal end of the supply pipe 30 is connected to the tube 31 outside the first tubular member 12 and is communicated with the refrigerant supply source 34 through the tube 31. The supply pipe 30 is a passage through which a refrigerant supplied through the tube 31 is supplied to the space 26.

The discharge pipe 32 is constituted of metal and is arranged along the axial direction inside the guide unit 40. A distal end of the discharge pipe 32 penetrates the proximal portion 25 of the second tubular member 22 and is communicated with the space 26. The discharge pipe 32 and the proximal portion 25 of the second tubular member 22 are joined. A proximal end of the discharge pipe 32 is connected to the tube 33 outside the first tubular member 12 and is communicated with the refrigerant accommodating container 36 through the tube 33. The discharge pipe 32 is a passage through which the refrigerant is discharged from the space 26.

The refrigerant supply source 34 accommodates the refrigerant used in the cooling device 10. As the refrigerant supply source 34, for example, a cold spray may be used. The cold spray retains compressed refrigerant gas therein and is configured to spray the refrigerant gas when a button thereof is pressed. Since the refrigerant gas is compressed within the spray, the refrigerant gas gasifies when sprayed out from the spray. The refrigerant supply source 34 is connected to the tube 31 and communicates with the space 26 through the tube 31 and the supply pipe 30. An inner diameter of the tube 31 and an inner diameter of the supply pipe 30 are set to be substantially identical to an inner diameter of a nozzle of the cold spray, and are configured relatively small. Due to this, the refrigerant maintains its compressed state while passing through the tube 31 and the supply pipe 30. The space 26 is broader than the tube 31 and the supply pipe 30. Due to this, the refrigerant gasifies in the space 26. Cooling agent in the cold spray is not particularly limited. For example, various types of gases, such as LP gas and CO₂ gas, can be liquified and used therein, however, use of the CO₂ gas is preferable for a surgery on human body such as endoscopic surgery. This is because CO₂ gas is introduced into a body during the endoscopic surgery. Thus, using a cold spray that uses CO₂ is preferable because it is safe even if the cooling gas leaks inside the body.

The refrigerant accommodating container 36 is connected to the tube 33 and communicates with the space 26 through the tube 33 and the discharge pipe 32. Since the space 26 communicates with the supply pipe 30 and the discharge pipe 32, when the refrigerant is supplied from the refrigerant supply source 34 to the space 26, the refrigerant in the space 26 is discharged to the refrigerant accommodating container 36 through the discharge pipe 32 and the tube 33. That is, the refrigerant is supplied from the refrigerant supply source 34, passes through the tube 31, the supply pipe 30, the space 26, the discharge pipe 32, and the tube 33, and is discharged to the refrigerant accommodating container 36.

The gripping forceps 60 are arranged inside the first tubular member 12. As shown in FIG. 3, the gripping forceps 60 include a pair of grip portions 61, a link mechanism 62 coupled to the pair of grip portions 61, and a rod 63 coupled to the link mechanism 62. Further, the gripping forceps 60 include a tube 64 covering the rod 63 and a handle 65 coupled to the rod 63 (see FIG. 1).

The pair of grip portions 61 is provided at a distal end portion of the gripping forceps 60. The pair of grip portions 61 faces each other and is configured to be capable of opening and closing. The pair of grip portions 61 opens and closes in a state where their grip surfaces 66 face each other. When the pair of grip portions 61 is closed, the suture needle 70 is held between the pair of grip portions 61.

The link mechanism 62 is provided between the pair of grip portions 61 and the rod 63. The link mechanism 62 connects the grip portions 61 and the rod 63. The link mechanism 62 transmits motion of the rod 63 to the pair of grip portions 61. The link mechanism 62 converts translational motion of the rod 63 to rotary motion of the grip portions 61. The pair of grip portions 61 opens and closes by rotating via the link mechanism 62.

The rod 63 is provided between the link mechanism 62 and the handle 65. The rod 63 connects the link mechanism 62 and the handle The rod translated in the axial direction by operation of the handle 65. The rod is arranged inside the tube 64. The tube 64 extends along the rod 63.

The handle 65 is provided at a proximal end portion of the gripping forceps 60. When the handle 65 is operated, the rod 63 is translated. Due to this, the pair of grip portions 61 opens and closes.

The gripping forceps 60 can be accommodated into the first tubular member 12 by being moved toward the proximal side in a state where the pair of grip portions 61 are closed. By moving the grip portions 61 to the proximal end of the first tubular member 12, the gripping forceps 60 can be detached from the cooling device 10. The gripping forceps 60 may be used in a state of being attached to the cooling device 10, or may be used separately in a state of being detached from the cooling device 10.

Next, an operation of the cooling device 10 will be described. To cool the suture needle 70 by using the cooling device 10, the gripping forceps 60 are brought to the state where the pair of grip portions 61 are closed, and are inserted into the first tubular member 12. Then, a position of the gripping forceps 60 is adjusted relative to the first tubular member 12 so that the grip portions 61 are positioned at the cooling unit 20 of the first tubular member 12. After the grip portions 61 have been positioned at the cooling unit 20, the refrigerant is then supplied from the refrigerant supply source 34 to the space 26. The refrigerant supplied from the refrigerant supply source 34 passes through the tube 31 and the supply pipe 30, and is supplied to the space 26. The refrigerant having been supplied to the gasifies in the space 26, and the gasified refrigerant is accommodated in the space 26. The space 26 communicates with the supply pipe 30 and the discharge pipe 32. Due to this, when the refrigerant is supplied from the supply pipe 30 to the space 26, the refrigerant accommodated in the space 26 passes through the discharge pipe 32 and the tube 33 and is discharged to the refrigerant accommodating container 36. The passages of the refrigerant in the cooling device 10 are sealed, and thus the refrigerant will not leak to outside of the cooling device 10.

When the refrigerant is supplied to the space 26, the cooling unit 20 is cooled. Specifically, the tube 14 and the second tubular member 22 in contact with the space 26 are cooled. Here, the tube 14 is constituted of the heat insulating material, while the second tubular member 22 is constituted of the metal with a high thermal conductivity. Due to this, the second tubular member 22 is cooled more intensively and a temperature thereof drops. As aforementioned, the inner peripheral surface of the second tubular member 22 is in contact with or close to the grip portions 61 of the gripping forceps 60, thus the grip portions 61 of the gripping forceps 60 are cooled via the second tubular member 22. When the grip portions 61 are cooled, the gripping forceps 60 are operated such that the grip portions 61 project from the distal end of the first tubular member 12. Then, the suture needle 70 is held by the grip portions 61, by which the suture needle 70 held by the gripping forceps 60 can be cooled. In a case where the suture needle 70 needs to be further cooled, the gripping forceps 60 may be further operated to make the suture needle 70 directly contact with the second tubular member 22 to cool the suture needle 70. The cooling device 10 can cool the suture needle 70 without making the refrigerant directly contact with the suture needle 70.

Next, the suture needle 70, which is an example of a shape-memory alloy member to be cooled by using the cooling device 10, will be described. The suture needle 70 is configured to suture an organ and tissue in a patient's body and is used in an endoscopic surgery. A suture thread is connected to the suture needle 70. The suture needle 70 is constituted of shape memory alloy. As the shape memory alloy, for example, Ni-Ti alloy, Cu-Zn-Al alloy, and Ni-Ti-Cu alloy may be exemplified. The shape memory alloy has a shape memory effect or super elasticity, and its shape changes by phase transformation or reverse transformation between an austenite phase and a martensite phase. The suture needle 70 constituted of the shape memory alloy has an arc shape as its original shape. Due to the characteristic of the shape memory alloy, when the suture needle 70 reaches a predetermined temperature or lower (martensite transformation point temperature Ms point or lower), its Young's modulus starts to decrease, and it becomes deformable when reaching a temperature of a Mf point (martensite transformation terminating temperature) or lower. On the other hand, the suture needle 70 starts to be restored from its deformed shape to the originally "memorized" arc shape when reaching a predetermined temperature or higher (austenite transformation point temperature As point or higher), and the Young's modulus increases at an Af point (austenite transformation terminating temperature) or higher and the suture needle 70 thus returns to its original shape and enters a super-elastic state. For example, in a case of using Ni-Ti alloy as a material of the suture needle 70, values of the respective transformation points (predetermined temperatures) can be adjusted by adjusting a ratio of Ni and Ti and by memory thermal treatment temperature. The predetermined temperature may be set by taking a human body temperature into consideration. For example, the predetermined temperature may be set in a range of 30 to 40°C, and the predetermined temperature of the present embodiment is 35°C (i.e., the body temperature). Further, in general, the temperature in a patient's body is higher than a temperature outside the body, and the predetermined temperature (e.g., 35°C) or higher is achieved inside the body, and the outside of the body is at the predetermined temperature or lower. Due to this, the suture needle 70 is at the predetermined temperature or lower when it is outside the patient's body, and thus is deformable. On the other hand, the suture needle 70 is at the predetermined temperature or higher when it is within the patient's body, and thus is restored to the original arc shape. Further, within the patient's body, the suture needle 70 is cooled to reach the predetermined temperature or lower. Due to this, the suture needle 70 comes to be at the predetermined temperature or lower, and thus becomes deformable. For example, in a case where the predetermined temperature is 35°C, when the suture needle 70 is in an environment with 35°C or lower outside the patient's body, its Young's modulus decreases and thus it is easily deformed or is freely deformable. On the other hand, when the suture needle 70 is introduced into the patient's body having the body temperature of 35°C or higher, it experiences reverse transformation and is restored to the original arc shape. The patient's body can be sutured by the suture needle 70 returning to its arc shape. Further, after the suture, the suture needle 70 is cooled to reach the transformation point Mf or lower, and thus it comes to be freely deformable. By deforming the suture needle 70, the suture needle 70 can easily be taken out from the patient's body.

Next, a method of using the suture needle with the above configuration will be described. As an example, a method of using the suture needle 70 in a laparoscopic surgery will be described. The laparoscopic surgery is a known surgery method, and is a surgery performed on an abdomen of a patient by creating a small incision in the abdomen of the patient instead of radically opening the abdomen of the patient, and is performed by inserting a laparoscope and another surgical instrument to an abdominal cavity of the patient.

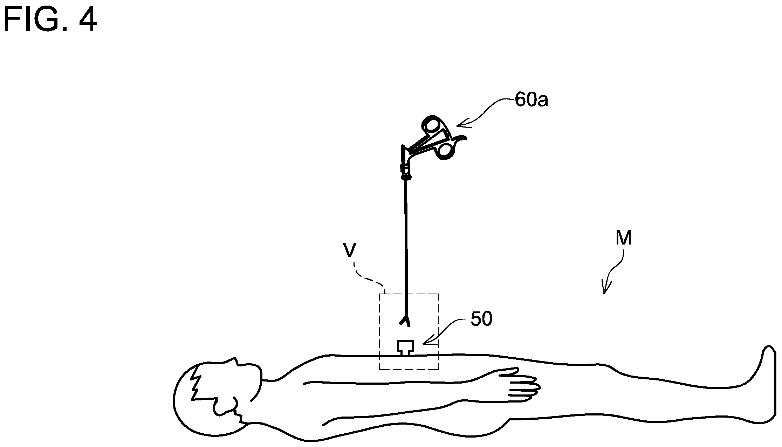

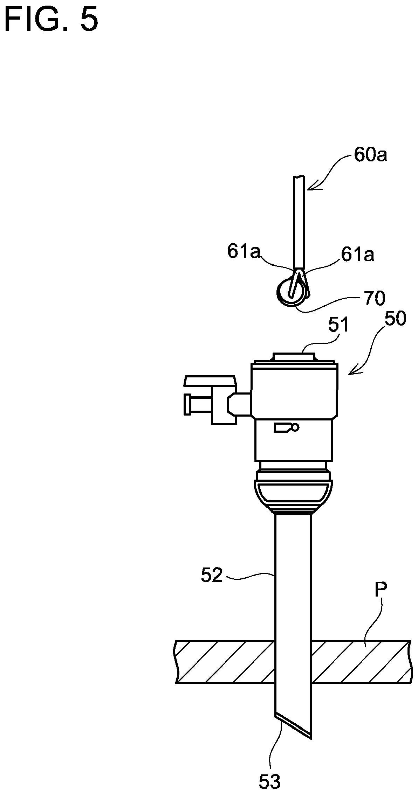

Upon performing the laparoscopic surgery, as shown in FIG. 4, a trocar 50 is inserted to an abdomen of a patient M. The trocar 50 is inserted to an incision created on the abdomen of the patient M. The trocar 50 is a known surgical instrument, and is an instrument for communicating inside and outside of the abdomen of the patient (inside and outside of the body) via the incision created on the abdomen of the patient M upon inserting the laparoscope and other surgical instrument to the abdominal cavity of the patient M. As shown in FIG. 5, the trocar 50 includes an inlet 51, a guide tube 52, and an outlet When the trocar 50 is installed in the abdomen P of the patient, the inlet 51 is open at the outside of the abdomen P of the patient (outside of the body), and the outlet 53 is open at the inside of the abdomen P of the patient (inside of the body). The inlet 51 and the outlet 53 of the trocar 50 communicate with inside of the guide tube 52. The guide tube 52 extends from the outside to the inside of the abdomen P of the patient (from the outside to the inside of the body).

When the suture needle 70 is used, the arc shape of the suture needle 70 is changed outside the abdomen P of the patient (at the outside of the body). Since the outside of the abdomen P of the patient (outside of the body) is at the predetermined temperature or lower, the suture needle 70 can be deformed. In the example shown in FIG. 5, the arc-shaped suture needle 70 is deformed to a ring shape. When the suture needle 70 is deformed outside the patient's body, it is preferable to bend the suture needle 70 to be in a shape in which a distal end thereof is curled inward. By so doing, the distal end of the suture needle 70 will not be caught by the inside of the guide tube 52 and within the patient's body.

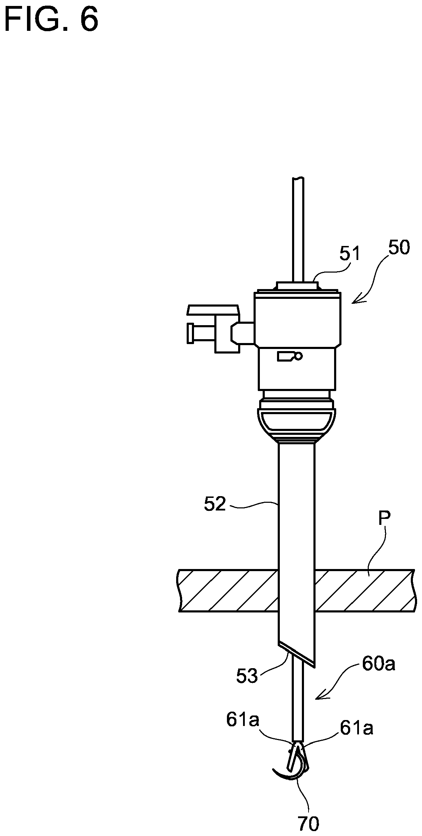

Next, the suture needle 70 is held by gripping forceps 60a in the state where the suture needle 70 has been deformed to the ring shape, and this suture needle 70 is inserted to the inside of the guide tube 52 from the inlet 51 of the trocar 50. The gripping forceps 60a are used separately, not in the state of being attached to the cooling device 10. As the gripping forceps 60a and the suture needle 70 are brought deeper into the guide tube 52, the gripping forceps 60a and the suture needle 70 are guided to the outlet 53 along the guide tube 52. Then, as shown in FIG. 6, the gripping forceps 60a and the suture needle 70 are delivered into the abdominal cavity of the patient (inside the body) from the outlet 53. When the suture needle 70 is delivered out to the inside of the abdomen P of the patient (inside the body), the suture needle 70 returns to its original arc shape as shown in FIG. 6. That is, the inside of the abdomen P of the patient (inside the body) is at the predetermined temperature or higher, so the suture needle 70 is restored to the original arc shape. After the suture needle 70 has been restored to the original arc shape, the patient's body is sutured by the suture needle 70. For example, an organ such as stomach or bowel is sutured.

After the suture, the suture needle 70 is cooled by using the cooling device 10. Specifically, the cooling device 10 is inserted to the abdomen P of the patient (inside the body). At this occasion, the cooling device 10 is inserted from an incision which is provided at a position different from that of the incision from which the gripping forceps 60a were inserted. By inserting the cooling device 10 from the incision which is different from the incision from which the gripping forceps 60a were inserted, the cooling device 10 can be inserted while the suture needle 70 is maintained to be held by the gripping forceps 60a. After this, as aforementioned, the grip portions 61 of the gripping forceps 60 attached to the cooling device 10 are cooled, after which the suture needle 70 held by the gripping forceps 60a is held by the grip portions 61 of the gripping forceps 60 attached to the cooing device 10.

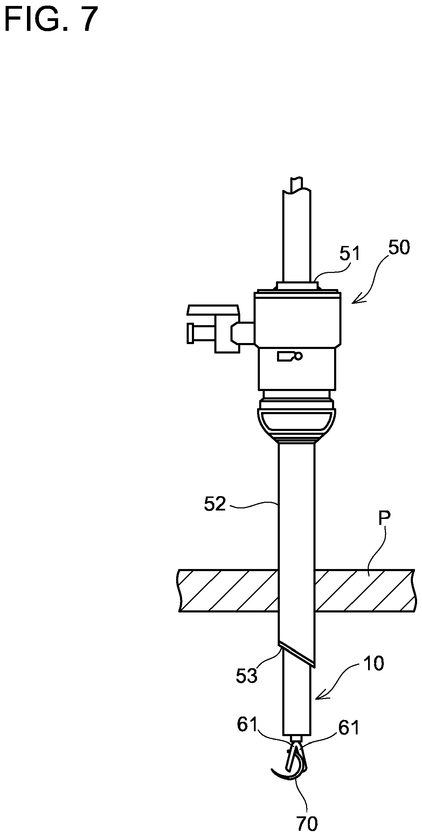

As shown in FIG. 7, when the suture needle 70 is held by the grip portions 61 of the gripping forceps 60 attached to the cooing device 10, the suture needle 70 is in the arc shape. Since the grip portions 61 are cooled by the cooling device 10, the suture needle 70 held by the grip portions 61 is also cooled and the temperature thereof drops, and thus it becomes deformable in a vicinity of a region thereof held by the grip portions 61. Due to this, the gripping forceps 60 and the cooling device 10 are taken out to outside the body from the trocar 50 as an integrated component. At this occasion, since the suture needle 70 is still in the arc shape, it makes contact with the inner side of the guide pipe 52 of the trocar 50. However, since the suture needle 70 is deformable at the region held by the grip portions 61, it deforms in this region. Due to this, the suture needle 70 can be taken out to outside of the body by passing through the guide pipe 52 of the trocar 50.

In this embodiment, the suture needle 70 constituted of the shape memory alloy can easily be cooled and deformed prior to its use, since it is outside the patient's body. Further, by setting the transformation point at 35°C, the suture needle 70 can easily be restored to its original shape inside the patient's body. In addition to this, by using the cooling device 10 of the present embodiment, the suture needle 70 can easily be cooled even when the suture needle 70 is located within the patient's body. Especially, the cooling device 10 can efficiently cool the cooling unit 20 without making the refrigerant directly contact with the inside of the patient's body. Due to this, the cooling device 10 can cool the suture needle 70 safely and promptly inside the patient's body.

Here, corresponding relationships between the aforementioned embodiment and the recitations in the claims will be described. The supply pipe 30 and the tube 31 are examples of "refrigerant supply pipe", the discharge pipe 32 and the tube 33 are examples of "refrigerant discharge pipe", the refrigerant accommodating container 36 is an example of "first refrigerant accommodating space", the inner peripheral surface of the second tubular member 22 is an example of "cooling face", the space 26 is an example of "second refrigerant accommodating space", and the proximal portion 16 of the tube 14 is an example of "guide pipe".

The cooling device 10 of the aforementioned embodiment is configured to cool the suture needle 70 used in the endoscopic surgery, however, a shape-memory alloy member to be a cooling target is not limited to the suture needle 70. For example, the cooling device 10 may be used to cool another medical instrument constituted of shape memory alloy and used in the endoscopic surgery. Further, instead of cooling the shape-memory alloy member by using the gripping forceps 60, the shape-memory alloy member may be brought to direct contact with the distal end of the cooling device.

Further, the present embodiment uses the cold spray as the refrigerant supply source 34, however, refrigerant other than the cold spray may be used as the refrigerant supply source 34. That is, the refrigerant may be any substance that exhibits a cooling effect, and may be, for example, liquid such as cold water. In the aforementioned embodiment, the refrigerant discharged from the space 26 is accommodated in the refrigerant accommodating container 36, however, no limitation is made to such a configuration. For example, in a case of using a highly safe substance as the refrigerant, the refrigerant may not be collected and may be directly discharged to outside of the cooling device 10.

Further, the cooling device 10 of the present embodiment is used to cool a medical instrument constituted of shape memory alloy, however, no limitation is made to such a configuration. For example, the cooling device 10 may be used to cool a shape-memory alloy member (such as an actuator) arranged inside a mechanical device. By using the cooling device disclosed herein, the shape-memory alloy member can be cooled without allowing the refrigerant to scatter inside the mechanical device. Further, by using flexible refrigerant supply pipe and refrigerant discharge pipe in the cooling device disclosed herein, pipe arrangement inside the mechanical device can be done relatively freely. In this case, by arranging the cooling unit at a position in contact with or close to the shape-memory alloy member, the shape-memory alloy member provided in the mechanical device can suitably be cooled.

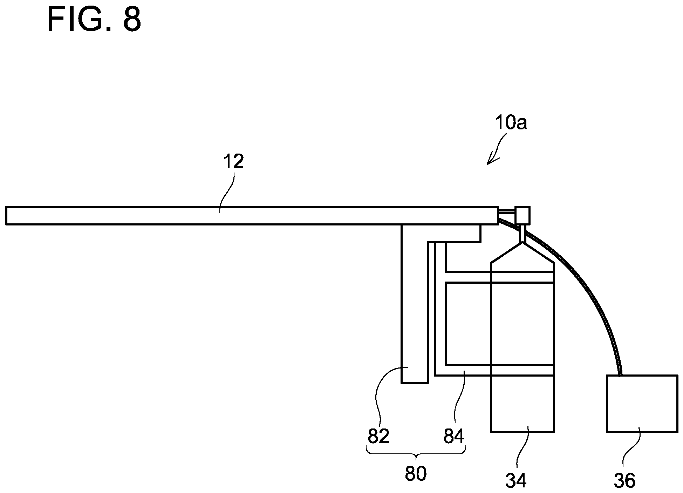

Further, as shown in FIG. 8, a cooling device 10a may include a handle 80 configured to control the refrigerant supplied from the refrigerant supply source 34. The cooling device 10a shown in FIG. 8 differs from the cooling device 10 in that it includes the handle 80, and is substantially identical to the cooling device 10 regarding other configurations. Thus, explanations for the configurations identical to those of the cooling device 10 will be omitted.

The handle 80 includes a support 82 and a holder 84. The support 82 is fixed to the first tubular member 12. The holder 84 retains the refrigerant supply source 34 and is arranged to be slidable relative to the support 82. When the holder 84 is separated away from the support 82, it is a state where the refrigerant is not supplied from the refrigerant supply source 34. When the holder 84 is slid and brought closer to the support 82, the refrigerant is supplied from the refrigerant supply source 34. As above, by adjusting a distance between the support 82 and the holder 84, the state where the refrigerant is supplied from the refrigerant supply source 34 and the state where the refrigerant is not supplied therefrom can be switched. That is, by using the handle 80, switching of the refrigerant supply states from the refrigerant supply source 34 and a timing to supply the refrigerant from the refrigerant supply source 34 can easily be adjusted.

Specific examples of the disclosure herein have been described in detail, however, these are mere exemplary indications and thus do not limit the scope of the claims. The art described in the claims includes modifications and variations of the specific examples presented above. Technical features described in the description and the drawings may technically be useful alone or in various combinations, and are not limited to the combinations as originally claimed.

## Claims

1. A cooling device configured to cool a shape-memory alloy member, the cooling device comprising:
a cooling unit configured to cool the shape-memory alloy member or a part that is to make contact with the shape-memory alloy member;
a refrigerant supply pipe including one end connected to a refrigerant supply source and other end connected to the cooling unit, the refrigerant supply pipe configured to supply a refrigerant supplied from the refrigerant supply source to the cooling unit; and
a refrigerant discharge pipe including one end connected to the cooling unit, the refrigerant discharge pipe configured to discharge the refrigerant discharged from the cooling unit from other end of the refrigerant discharge pipe.

2. The cooling device according to claim 1, further comprising:
a first refrigerant accommodating space connected to the other end of the refrigerant discharge pipe,
wherein the first refrigerant accommodating space is configured to accommodate the refrigerant discharged from the refrigerant discharge pipe.

3. The cooling device according to claim 1 or 2, wherein
the cooling unit comprises:
a cooling surface configured to cool the shape-memory alloy member or a part that is to make contact with the shape-memory alloy member; and
a second refrigerant accommodating space configured to accommodate the refrigerant for cooling the cooling surface,
wherein the refrigerant supplied from the refrigerant supply source is supplied to the second refrigerant accommodating space through the refrigerant supply pipe, and the refrigerant supplied to the second refrigerant accommodating space is discharged through the refrigerant discharge pipe.

4. The cooling device according to claim 3, wherein
the second refrigerant accommodating space is covered with a heat insulating material excluding a cooling surface side thereof.

5. The cooling device according to claim 3 or 4, wherein
the cooling surface is constituted of copper, copper alloy, aluminum, aluminum alloy, or metal having higher thermal conductivity than aluminum.

6. The cooling device according to any one of claims to further comprising:
an operation handle configured to control a supply state and a non-supply state, the supply state being a state where the refrigerant is supplied from the refrigerant supply source to the second refrigerant accommodating space, and the non-supply state being a state where the refrigerant is not supplied from the refrigerant supply source to the second refrigerant accommodating space.

7. The cooling device according to any one of claims to further comprising:
a guide pipe configured to accommodate the refrigerant supply pipe and the refrigerant discharge pipe, and to guide the refrigerant supply pipe and the refrigerant discharge pipe to the cooling unit,
wherein the cooling unit comprises a tubular member connected to one end of the guide pipe, the tubular member provided with the cooling surface on a surface thereof and provided with the second refrigerant accommodating space inside thereof.
